# EUROPEAN PATENT APPLICATION

(11) **EP 4 635 340 A1**
(43) Date of publication of application: **22.10.2025**
(21) Application number: 22968556.5
(22) Date of filing: 16.12.2022
(51) Int. Cl.: A24F 40/57, A24F 40/53, A24F 40/85

(54) **INHALATION DEVICE, CONTROL METHOD, AND PROGRAM**

(71) Applicant: Japan Tobacco Inc., Tokyo 105-6927 (JP)
(72) Inventor: FUJIKI, Takashi, Tokyo 130-8603 (JP)
(74) Representative: Hoffmann Eitle
(86) International application number: PCT/JP2022/046464
(87) International publication number: WO 2024/127647

(57) **Abstract**

An MCU (1) of an inhalation device (100) operates a heating unit (121C) based on a stick heating profile for heating a stick-type substrate (150) when the stick-type substrate (150) is accommodated in an accommodating portion (140C), and operates the heating unit (121C) based on a cleaning heating profile different from the stick heating profile when the stick-type substrate (150) is not accommodated in the accommodating portion (140C).

## Description

### TECHNICAL FIELD

The present disclosure relates to an inhalation device, a control method, and a program for generating aerosol from a substrate having an aerosol source.

### BACKGROUND ART

Inhalation devices that generate an aerosol with added flavor components and allow a user to inhale the generated aerosol, for example, are conventionally known. Such inhalation devices typically deliver aerosol to the user by heating a substrate containing an aerosol source with a heating unit (also referred to as a "heating element") that is an electric resistance heater or an induction heater.

The accommodating portion into which the substrate is inserted may become dirty with use of the inhalation device. Therefore, conventional inhalation devices have included ones that allow the accommodating portion to be cleaned periodically using cleaning tools with cleaning agents such as alcohol or water. For example, PTL 1 discloses cleaning the interior of a cavity by inserting a cleaning article (such as a brush) into the cavity or adding a cleaning component.

### CITATION LIST

### PATENT LITERATURE

[PTL 1] JP 2012-513750 A

### SUMMARY OF INVENTION

### TECHNICAL PROBLEM

When the accommodating portion is cleaned using cleaning tools with cleaning agents such as alcohol or water, the interior of the accommodating portion may become wet immediately after cleaning. If the user uses the inhalation device while the interior of the accommodating portion is wet, poor-quality smoking flavor aerosol may be delivered to the user. Therefore, in the prior art, to obtain a high-quality smoking experience, the user needed to wait for the interior of the accommodating portion to dry naturally after cleaning, which was inconvenient.

The present disclosure provides an inhalation device, a control method, and a program that improve the convenience of using the inhalation device.

### SOLUTION TO PROBLEM

One aspect of the present disclosure is an inhalation device for generating aerosol from a substrate having an aerosol source, comprising an accommodating portion in which the substrate is accommodated, a heating unit for heating the accommodating portion, and a control unit for controlling the heating unit based on heating information that defines the time-series transition of a target temperature, which is the target value of a temperature of the heating unit, wherein the heating information includes first heating information for heating the substrate and second heating information different from the first heating information, and the control unit operates the heating unit based on the first heating information when the substrate is accommodated in the accommodating portion, and operates the heating unit based on the second heating information when the substrate is not accommodated in the accommodating portion.

Another aspect of the present disclosure is a control method executed by a computer for controlling the operation of an inhalation device that generates aerosol from a substrate having an aerosol source, wherein the inhalation device includes an accommodating portion in which the substrate is accommodated and a heating unit for heating the accommodating portion, the computer is configured to be able to control the heating unit based on heating information that defines the time-series transition of a target temperature which is the target value of a temperature of the heating unit, the heating information includes first heating information for heating the substrate and second heating information different from the first heating information, and the computer operates the heating unit based on the first heating information when the substrate is accommodated in the accommodating portion, and operates the heating unit based on the second heating information when the substrate is not accommodated in the accommodating portion.

Another aspect of the present disclosure is a program that causes a computer that controls operation of an inhalation device that generates aerosol from a substrate having an aerosol source to execute predetermined processing, wherein the inhalation device includes an accommodating portion in which the substrate is accommodated and a heating unit for heating the accommodating portion, the computer is configured to control the heating unit based on heating information that defines the time-series transition of a target temperature which is the target value of a temperature of the heating unit, the heating information includes first heating information for heating the substrate and second heating information different from the first heating information, and the program the program causes the computer to execute processing for operating the heating unit based on the first heating information when the substrate is accommodated in the accommodating portion, and operating the heating unit based on the second heating information when the substrate is not accommodated in the accommodating portion.

### ADVANTAGEOUS EFFECTS OF INVENTION

According to the present disclosure, the convenience of using the inhalation device can be improved.

### BRIEF DESCRIPTION OF DRAWINGS

Figure 1 is a schematic diagram showing a first configuration example (inhalation device 100A) of the inhalation device.
Figure 2 is a schematic diagram showing a second configuration example (inhalation device 100B) of the inhalation device.
Figure 3 is an overall perspective view of the inhalation device 100, which is an embodiment of the present disclosure.
Figure 4 is a perspective view of the internal unit 10 viewed from the right front.
Figure 5 is a perspective view of the internal unit 10 viewed from the left front.
Figure 6 is an exploded perspective view of the internal unit 10.
Figure 7 is a cross-sectional perspective view of the heater assembly 30.
Figure 8 is a cross-sectional view along line A-A in Figure 5, showing the structure around the sensor FPC 73, stick detection sensor 12, and stick guide 31 (accommodating portion 140C).
Figure 9 is a schematic diagram showing the path of light emitted from the stick detection sensor 12 in the accommodated and non-accommodated states of the stick-type substrate 150.
Figure 10 is a graph showing the detection or non-detection of the stick-type substrate 150 based on brightness.
Figure 11 is a graph showing the heating profile for the stick and the cleaning heating profile.
Figure 12 is a flowchart showing an example of processing executed by the MCU 1.
Figure 13 is a graph explaining the regions (first region to third region) of the brightness of reflected light detected by the stick detection sensor 12.

### DESCRIPTION OF EMBODIMENTS

Hereinafter, the inhalation device, control method, and program according to an embodiment of the present disclosure will be described with reference to the drawings. First, two configuration examples (first configuration example and second configuration example) to which the configuration of the inhalation device of the present disclosure can be applied will be described. Note that in the following, the same or similar elements may be denoted by the same or similar reference numerals, and their descriptions may be omitted or simplified as appropriate.

### <<1. Configuration Example of Inhalation Device>>

An inhalation device is a device for generating a substance to be inhaled by a user. Hereinafter, the substance generated by the inhalation device will be described as being an aerosol. Alternatively, the substance generated by the inhalation device may be a gas.

### (1) First Configuration Example

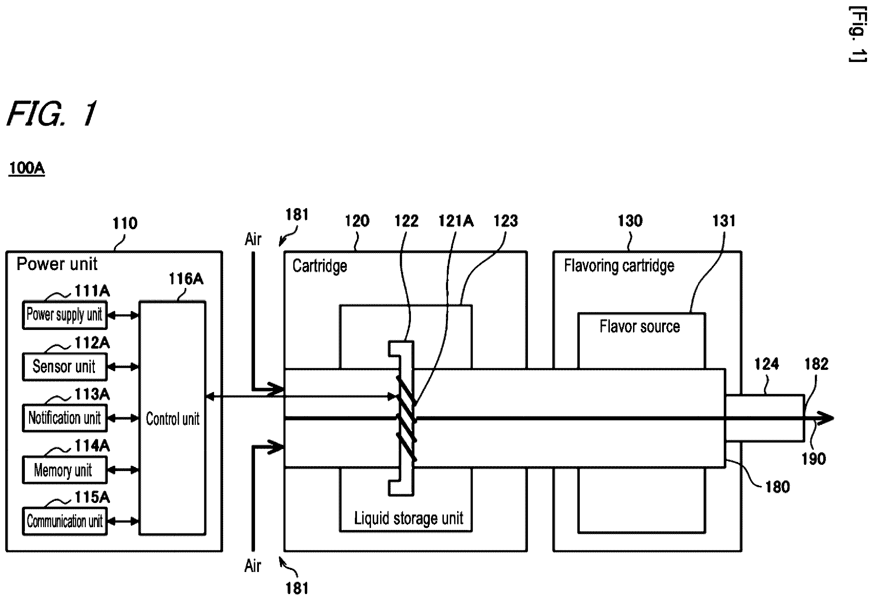

Figure 1 is a schematic diagram showing a first configuration example of the inhalation device. As shown in Figure 1, the inhalation device 100A according to this configuration example includes a power unit 110, a cartridge 120, and a flavoring cartridge 130. The power unit 110 comprises a power supply unit 111A, a sensor unit 112A, a notification unit 113A, a memory unit 114A, a communication unit 115A, and a control unit 116A. The cartridge 120 includes a heating unit 121A, a liquid guiding portion 122, and a liquid storage portion 123. The flavoring cartridge 130 includes a flavor source 131 and a mouthpiece 124. An air flow path 180 is formed in the cartridge 120 and the flavoring cartridge 130.

The power supply unit 111A stores electrical power. The power supply unit 111A then supplies the electric power to each component of the inhalation device 100A in accordance with control performed by the control unit 116A. The power supply unit 111A may be configured, for example, by a rechargeable battery such as a lithium ion secondary battery.

The sensor unit 112A acquires various types of information relating to the inhalation device 100A. As an example, the sensor unit 112A is configured by a pressure sensor such as a condenser microphone, a flow rate sensor or a temperature sensor, etc., and acquires values associated with inhalation by a user. As another example, the sensor unit 112A is configured by an input device, such as a button or switch, for accepting input of information from the user.

The notification unit 113A notifies the user of the information. The information notified by the notification unit 113A includes, for example, various information such as the State Of Charge (SOC) indicating the charging state of the power supply unit 111A, preheating time during inhalation, and inhalation possible period. The notification unit 113A can be configured by a light-emitting device which emits light, a display device which displays images, a sound output device which outputs sound, or a vibration device which vibrates, etc., for example.

The memory unit 114A stores various information for the operation of the inhalation device 100A. The memory unit 114A can be configured by a non-volatile storage medium such as a flash memory, for example.

The communication unit 115A is a communication interface capable of performing communication in accordance with any wired or wireless communication standard. Such communication standards may include, for example, Wi-Fi, Bluetooth, BLE (Bluetooth Low Energy), NFC (Near Field Communication), or LPWA (Low Power Wide Area).

The control unit 116A functions as an arithmetic processing device and control device, controlling the overall operation within the inhalation device 100A according to various programs. The control unit 116A is realized by an electronic circuit such as a CPU (Central Processing Unit) or a microprocessor.

The liquid storage portion 123 stores an aerosol source. The aerosol source is atomized so as to generate an aerosol. The aerosol source is a polyhydric alcohol such as glycerol or propylene glycol, or a liquid such as water, for example. The aerosol source may include tobacco-derived or non-tobacco-derived flavor components. If the inhalation device 100A is a medical inhaler such as a nebulizer, the aerosol source may include a drug.

The liquid guiding portion 122 guides the aerosol source, which is the liquid stored in the liquid storage portion 123, from the liquid storage portion 123, and holds the aerosol source. The liquid guiding portion 122 is, for example, a wick formed by twisting a fibrous material such as glass fibers or a porous material such as a porous ceramic. In such case, the aerosol source stored in the liquid store portion 123 is guided by the capillary effect of the wick.

The heating unit 121A heats the aerosol source to atomize the aerosol source, thereby generating the aerosol. In the example shown in Figure 1, the heating unit 121A is configured as a coil and is wound around the liquid guiding unit 122. When the heating unit 121A generates heat, the aerosol source held in the liquid guiding portion 122 is then heated and atomized, generating the aerosol. The heating unit 121A generates heat when powered by the power supply unit 111A. For example, power may be supplied to the heating unit 121A when it is detected by the sensor unit 112A that the user has started inhalation and/or predetermined information has been input. Then, when it is detected by the sensor unit 112A that the user has finished inhalation and/or predetermined information has been input, the power supply to the heating unit 121A may be stopped. The user's inhalation operation on the inhalation device 100A can be detected based on the internal pressure within the inhalation device 100A exceeding a predetermined threshold, for example, by a puff sensor.

The flavor source 131 is a component for imparting a flavor component to the aerosol. The flavor source 131 may include tobacco-derived or non-tobacco-derived flavor components.

The air flow path 180 is a flow path for air to be inhaled by the user. The air flow path 180 has a tubular structure with an air inflow hole 181, which is an inlet for air into the air flow path 180, and an air outflow hole 182, which is an outlet for air from the air flow path 180. Midway through the air flow path 180, the liquid guiding portion 122 is disposed upstream (closer to the air inflow hole 181), and the flavor source 131 is disposed downstream (closer to the air outflow hole 182). Air flowing in through the air inflow hole 181 upon inhalation by the user is mixed with the aerosol generated by the heating unit 121A and transported through the flavor source 131 to the air outlet hole 182, as shown by the arrow 190. When the mixed fluid of aerosol and air passes through the flavor source 131, the flavor component contained in the flavor source 131 is applied to the aerosol.

The mouthpiece 124 is a member which is held in the user's mouth during inhalation. The air outlet hole 182 is disposed in the mouthpiece 124. The user holds the mouthpiece 124 in their mouth to make it possible to draw the mixed fluid of aerosol and air into the oral cavity.

A configuration example of the inhalation device 100A has been described above. The inhalation device 100A is, of course, not limited to the configuration described above, and may adopt various configurations, such as those illustrated below by way of example.

As an example, the inhalation device 100A need not include the flavoring cartridge 130. In such case, the cartridge 120 is provided with the mouthpiece 124.

As another example, the inhalation device 100A may include a plurality of types of aerosol sources. Other types of aerosol may be generated by a plurality of types of aerosol generated from the plurality of types of aerosol sources being mixed in the air flow path 180 to cause a chemical reaction.

Furthermore, the means for atomizing the aerosol source is not limited to heating provided by the heating unit 121A. For example, the means for atomizing the aerosol source may be vibration atomization or induction heating.

### (2) Second Configuration Example

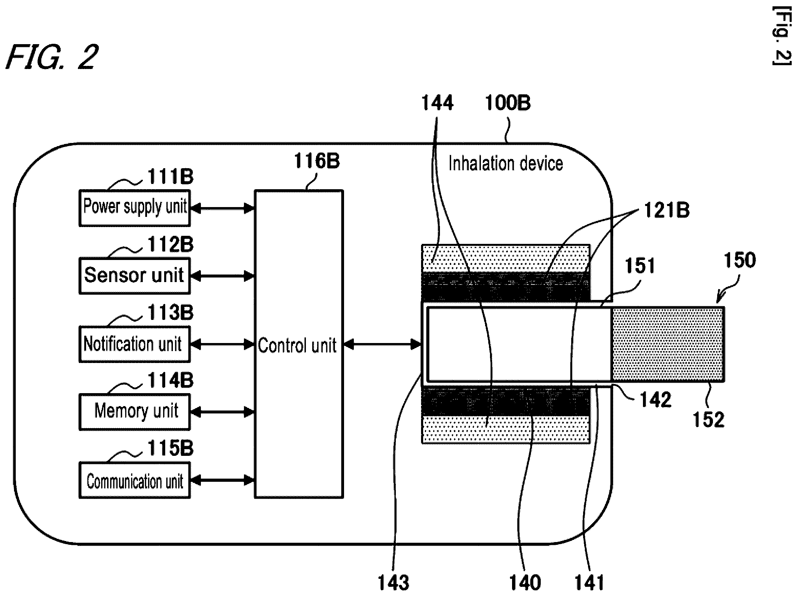

Figure 2 is a schematic diagram showing a second configuration example of the inhalation device. As shown in Figure 2, the inhalation device 100B according to this configuration example includes a power supply unit 111B, a sensor unit 112B, a notification unit 113B, a memory unit 114B, a communication unit 115B, a control unit 116B, a heating unit 121B, an accommodating portion 140, and a heat insulation unit 144. In the first configuration example, the inhalation device 100A had a separate power unit 110 housing the power supply unit 111A and the heating unit 121A, but in the second configuration example, the inhalation device 100B has the power supply unit 111B and the heating unit 121B integrated. That is, the inhalation device 100B in the second configuration example can also be referred to as a power supply unit with a built-in heating unit.

Each of the power supply unit 111B, sensor unit 112B, notification unit 113B, memory unit 114B, communication unit 115B, and control unit 116B is substantially the same as the corresponding component included in the inhalation device 100A of the first configuration example.

The accommodating portion 140 has an internal space 141 and holds the stick-type substrate 150 while accommodating a part of the stick-type substrate 150 in the internal space 141. The accommodating portion 140 has an opening 142 that communicates the internal space 141 with the outside, and accommodates the stick-type substrate 150 inserted into the internal space 141 from the opening 142. For example, the accommodating portion 140 is a cylindrical body with the opening 142 and the bottom 143 as the bottom surface, defining a columnar internal space 141. An air flow path for supplying air to the internal space 141 is connected to the accommodating portion 140. An air inflow hole, which is an inlet for air into the air flow path, is disposed in a side surface of the inhalation device 100, for example. An air outflow hole serving as an outlet for air from the air flow path to the internal space 141 is disposed in the bottom portion 143, for example.

The stick-type substrate 150 comprises a substrate portion 151 and a mouthpiece portion 152. The substrate section 151 includes an aerosol source. The aerosol source may include tobacco-derived or non-tobacco-derived flavor components. If the inhalation device 100B is a medical inhaler such as a nebulizer, the aerosol source may include a drug. The aerosol source may be, for example, a liquid such as water and polyhydric alcohols such as glycerol and propylene glycol comprising the tobacco-derived or non-tobacco-derived flavor component, or else may be a solid comprising the tobacco-derived or non-tobacco-derived flavor component. When the stick-type substrate 150 is held in the accommodating portion 140, at least a part of the substrate section 151 is accommodated in the internal space 141, and at least a part of the mouthpiece section 152 protrudes from the opening 142. When the user holds the mouthpiece section 152 protruding from the opening 142 in the mouth and inhales, air flows into the internal space 141 via an air flow path not shown in the figure, and reaches the user's mouth along with the aerosol generated from the substrate section 151.

In the example shown in Figure 2, the heating unit 121B is configured in a film shape and is arranged to cover the outer circumference of the accommodating portion 140. Then, when the heating units 121B generate heat, the substrate portion 151 of the stick-shaped substrate 150 is heated from the outer periphery, generating the aerosol.

The heat insulating portion 144 prevents heat transfer from the heating units 121B to other components. For example, the heat insulating portion 144 is configured from a vacuum heat insulating material or an aerogel heat insulating material, or the like.

A configuration example of the inhalation device 100B has been described above. The inhalation device 100B is, of course, not limited to the configuration described above, and may adopt various configurations, such as those illustrated below by way of example.

As an example, the heating unit 121B may be configured in a blade shape and arranged to protrude into the internal space 141 from the bottom 143 of the accommodating portion 140. In that case, the blade-like heating unit 121B is inserted into the substrate portion 151 of the stick-type substrate 150 and heats the substrate portion 151 of the stick-type substrate 150 from the inside. In another example, the heating unit 121B may be arranged to cover the bottom 143 of the accommodating portion 140. Additionally, the heating unit 121B may be configured as a combination of two or more of a first heating unit covering the outer circumference of the accommodating portion 140, a blade-shaped second heating unit, and a third heating unit covering the bottom 143 of the accommodating portion 140.

As another example, the accommodating portion 140 may comprise an opening/closing mechanism such as a hinge for opening/closing part of an outer shell that forms the internal space 141. By opening and closing the outer shell, the accommodating portion 140 may then receive and hold the stick-type substrate 150 which has been inserted into the internal space 141. In this case, the heating unit 121B may be provided at the holding location of the accommodating portion 140 and may heat the stick-type substrate 150 while pressing it.

Additionally, the means for atomizing the aerosol source is not limited to heating by the heating unit 121B. For example, the means for atomizing the aerosol source may be induction heating. In that case, the inhalation device 100B comprises at least an electromagnetic induction source such as a coil for generating a magnetic field, instead of the heating unit 121B. A susceptor which generates heat by means of induction heating may be provided in the inhalation device 100B, or may be contained in the stick-type substrate 150.

The inhalation device 100B may further include the heating unit 121A, the liquid guiding portion 122, the liquid storage portion 123, and the air flow path 180 according to the first configuration example, and the air flow path 180 may supply air to the internal space 141. In this case, the mixed fluid of aerosol and air generated by the heating unit 121A flows into the internal space 141 and is further mixed with the aerosol generated by the heating unit 121B to reach the oral cavity of the user.

### <<2. Configuration Example of the Inhalation Device of the Present Disclosure>>

Next, an embodiment of the inhalation device to which the configuration of the inhalation device of the present disclosure is applied to the inhalation device 100B of the second configuration example described above (hereinafter referred to as the inhalation device 100) will be described. Although specific descriptions are omitted, some of the configurations of the inhalation device 100 described in detail below can also be applied to the inhalation device 100A of the first configuration example.

### [Overall Configuration of the Inhalation Device]

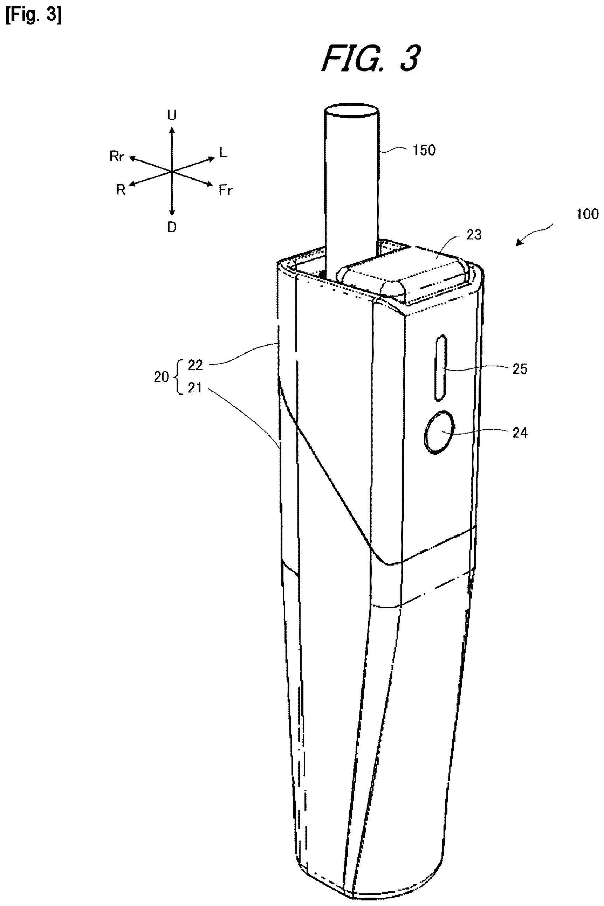

Figure 3 is an overall perspective view of the inhalation device 100. In the following, in the inhalation device 100, the insertion and removal direction of the stick-type substrate 150 relative to the inhalation device 100 is defined as the vertical direction, the sliding movement direction of the shutter 23 described later is defined as the front-rear direction, and the direction orthogonal to the vertical and front-rear directions is defined as the left-right direction. Also, as shown in the figure, the front is denoted as Fr, the rear as Rr, the left side as L, the right side as R, the upper side as U, and the lower side as D.

The inhalation device 100 is preferably sized to fit in the hand, for example, having a rod shape. For example, the user holds the inhalation device 100 with one hand while touching the surface of the inhalation device 100 with the fingertips. Note that the shape of the inhalation device 100 is not limited to a rod shape and can be any shape (e.g., a rounded rectangular shape or an egg shape).

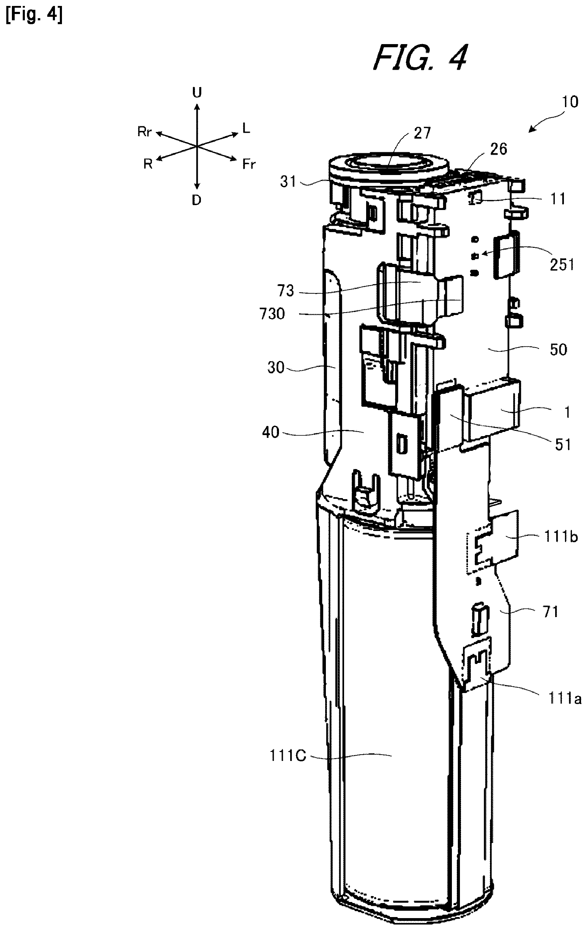

The inhalation device 100 includes an internal unit 10 (see Figures 4 to 6) and a case 20 that constitutes the appearance of the inhalation device 100. The case 20 includes a lower case 21 and an upper case 22. A part of the internal unit 10 is accommodated in the lower case 21, and the entire internal unit 10 is accommodated in the case 20 by covering the upper case 22 from above the lower case 21.

The upper surface of the inhalation device 100 is provided with an opening 27 (see Figures 4 to 6) for inserting and removing the stick-type substrate 150 and a shutter 23 that can slide in the front-rear direction. The opening 27 is arranged on the rear side of the upper surface of the inhalation device 100. The shutter 23 selectively takes an open state (front position) that allows the insertion and removal of the stick-type substrate 150 by opening the opening 27 and a closed state (rear position) that closes the opening 27 by positioning the shutter 23 above the opening 27. When inserting the stick-type substrate 150 into the opening 27, the user sets the shutter 23 to the open state.

A shutter detection sensor 11 (see Figure 4) is provided near the shutter 23. The shutter detection sensor 11 detects whether the shutter 23 is in the open state. The shutter detection sensor 11 is an example of the sensor unit 112B of the inhalation device 100B shown in Figure 2.

Additionally, the upper surface of the inhalation device 100 is provided with a USB (Universal Serial Bus) port 26 (see Figure 4) arranged adjacent to the opening 27. In the aforementioned open state, the shutter 23 closes the USB port 26. On the other hand, in the aforementioned closed state, the shutter 23 does not close the USB port 26, and the USB port 26 is open. The USB port 26 is configured to be electrically connectable to an external power source (not shown) capable of supplying power to charge the power supply unit 111C (see Figure 4). The USB port 26 is, for example, a receptacle into which a plug on the opposite side can be inserted. In this embodiment, for example, the USB port 26 is a USB Type-C shaped receptacle.

The front surface of the inhalation device 100 is provided with an operation unit 24 and a light-emitting unit 25. The operation unit 24 is arranged below the light-emitting unit 25. More specifically, the operation unit 24 and the light-emitting unit 25 are components of the internal unit 10 accommodated in the case 20, and a part of the operation unit 24 and the light-emitting unit 25 is configured to be exposed from an opening formed on the front surface of the case 20. The light-emitting unit 25 is an example of the notification unit 113B of the inhalation device 100B shown in Figure 2.

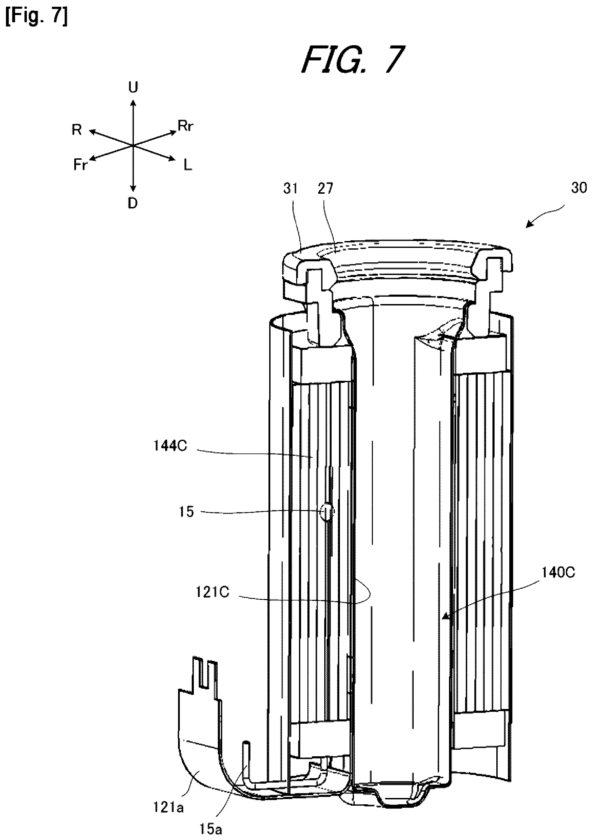

The operation unit 24 is a button-type switch operable by the user and is an input device that receives input of information from the user. The operation unit 24 is connected to the main board 50 (see Figures 4 to 6) described later. By pressing the operation unit 24, for example, the MCU (Micro Controller Unit) 1 (see Figures 4 to 6) and the heating unit 121C (see Figure 7) are activated. Note that the MCU 1 functions as the control unit 116B in the inhalation device 100B. In addition to functioning as the control unit 116B in the inhalation device 100B, the MCU 1 may also integrally have the function of the communication unit 115B. Furthermore, the MCU 1 may be composed of a single IC or two or more ICs. For example, discharge control to the heating unit 121C and charge control to the power supply unit 111C may be performed by a single IC or by separate ICs.

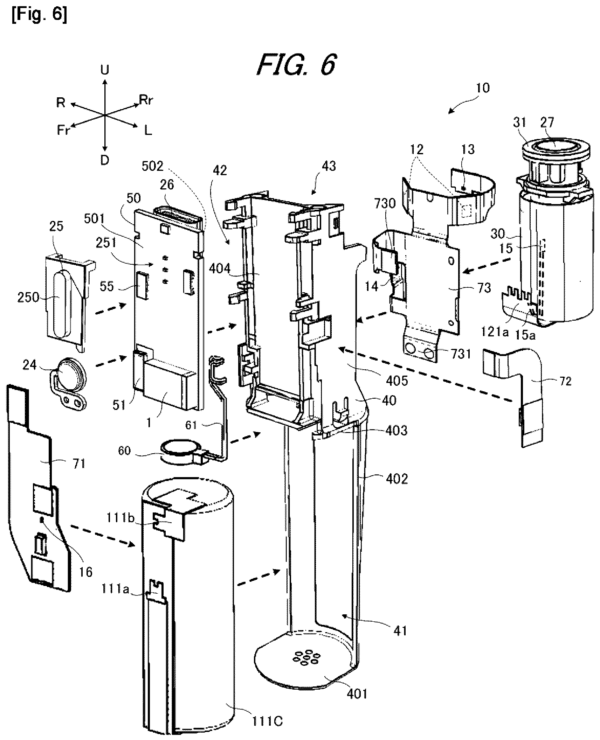

The light-emitting unit 25 comprises a light-emitting element such as an LED (Light Emitting Diode). More specifically, the light-emitting unit 25 includes multiple LEDs 251 (see Figure 6) provided on the main board 50 and a transparent cover 250 that covers the multiple LEDs 251 and transmits the light of the LEDs 251. A part of the transparent cover 250 is exposed from an opening formed on the front surface of the case 20. In this embodiment, for example, the multiple LEDs 251 are configured to emit light in multiple emission colors, including blue, yellow, and red. Note that the number of light-emitting elements can be set arbitrarily, and for example, the light-emitting unit 25 may have only one light-emitting element.

The light-emitting unit 25 emits light in a predetermined emission mode based on a command from the MCU 1 to notify the user of predetermined information. Here, the emission mode can be, for example, the emission color, but is not limited to this and can be, for example, the intensity of the lighting (in other words, brightness) or the lighting pattern (e.g., blinking at predetermined time intervals), etc. Additionally, the predetermined information is, for example, operation information indicating whether the power of the inhalation device 100 is on.

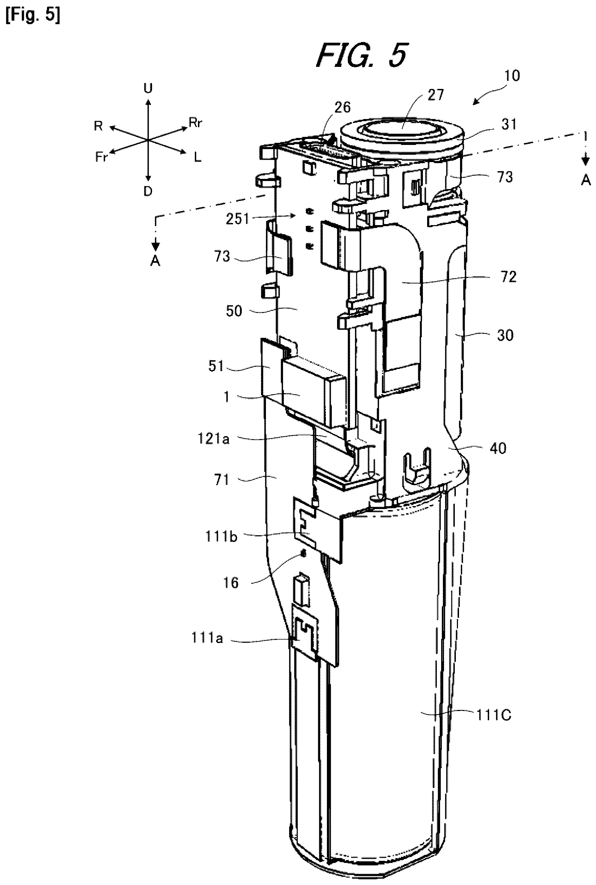

Next, the internal unit 10 of the inhalation device 100 of this embodiment will be described with reference to Figures 4 to 6. Figure 4 is a perspective view of the internal unit 10 viewed from the right front, Figure 5 is a perspective view of the internal unit 10 viewed from the left front, and Figure 6 is an exploded perspective view of the internal unit 10. Note that the internal unit 10 is the inhalation device 100 with the case 20 and the shutter 23 removed.

The internal unit 10 includes a chassis 40, a main board 50, a vibrating device 60, a heater assembly 30, a power supply unit 111C, a power board 71, a peripheral FPC (Flexible Printed Circuits) 72, a sensor FPC 73, and various sensors. Note that the power board 71 may be a flexible circuit board, a rigid board described later, or a combination of a flexible board and a rigid board, but here it is described as an example of a flexible circuit board.

### (Chassis)

As shown in the exploded perspective view of Figure 6, the chassis 40 includes a power holding section 41 that holds the power supply unit 111C, a board holding section 42 that holds the main board 50, and a heater holding section 43 that holds the heater assembly 30. The power holding section 41 is located at the lower part of the chassis 40, and the board holding section 42 and the heater holding section 43 are located at the upper part of the chassis 40.

The power holding section 41 has a cylindrical shape with a part of the side surface cut out, in other words, a substantially semi-cylindrical shape. The power holding section 41 includes a bottom wall section 401, a side wall section 402 that has an arc shape and stands upright from the bottom wall section 401, and an upper wall section 403 provided at the upper end of the side wall section 402. The power supply unit 111C is arranged in the space surrounded by the bottom wall section 401, the side wall section 402, and the upper wall section 403.

The board holding section 42 is provided on the standing wall section 404 that stands upright from the upper wall section 403 of the power holding section 41. The board holding section 42 is provided on one side (here, the front side) of the standing wall section 404 in the front-rear direction and holds the main board 50.

The heater holding section 43 is provided on the opposite side (here, the rear side) of the standing wall section 404 from the board holding section 42 in the front-rear direction. The heater holding section 43 has a space surrounded by the standing wall section 404, a pair of left and right wall sections 405 extending in the front-rear direction from the standing wall section 404, and the upper surface of the upper wall section 403 of the power holding section 41, and the heater assembly 30 is arranged in this space.

### (Main Board)

The main board 50 is a rigid board with multiple electronic components (elements) mounted on both sides. The main board 50 has the MCU 1, LEDs 251, a charging IC (Integrated Circuit), a boost DC/DC converter, etc., mounted on it. The main board 50 is held by the board holding section 42 of the chassis 40 so that the element mounting surface faces the front-rear direction. In Figure 6, only the front surface 501 (here, the front side) of the main board 50 is shown. Therefore, the charging IC and the boost DC/DC converter mounted on the rear surface 502 (here, the rear side) are not shown.

A power connection section 51 electrically connected to the power supply unit 111C is provided in the lower region of the front surface 501 of the main board 50. The power connection section 51 is electrically connected to the power supply unit 111C via the power board 71. The power supply unit 111C is a cylindrical lithium-ion secondary battery and is an example of the power supply unit 111B of the inhalation device 100B shown in Figure 2.

As shown in Figure 6, the power supply unit 111C is provided with a positive electrode tab 111a and a negative electrode tab 111b. The power supply unit 111C is arranged in the power holding section 41 of the chassis 40 so that the positive electrode tab 111a and the negative electrode tab 111b are positioned at the front. The power board 71 is positioned in front of the power supply unit 111C and the main board 50, extending in the vertical direction. The power board 71 is connected to the positive electrode tab 111a and the negative electrode tab 111b of the power supply unit 111C and is also connected to the power connection section 51 of the main board 50. The power from the power supply unit 111C is transmitted to the main board 50 through the conductive tracks formed on the power board 71 and supplied to each electronic component. Additionally, the power board 71 is equipped with a power temperature sensor 16. The power temperature sensor 16 is a sensor that detects the temperature of the power supply unit 111C. The power temperature sensor 16 is, for example, a thermistor. The power temperature sensor 16 is an example of the sensor unit 112B of the inhalation device 100B shown in Figure 2.

A USB port 26 is provided in the upper region of the rear surface 502 of the main board 50. The USB port 26 is electrically connected to a charging IC (not shown) via wiring formed on the main board 50.

In addition to the charging IC and the boost DC/DC converter (not shown), a heater connection section is provided on the rear surface 502 of the main board 50. The charging IC performs charging control to supply (charge) the power input from the USB port 26 to the power supply unit 111C. The boost DC/DC converter boosts the power supplied from the power supply unit 111C to generate power supplied to the heating unit 121C (see Figure 7).

The heater connection section is connected to the board connection section 121a extending from below the heater assembly 30, supplying power to the heating unit 121C of the heater assembly 30. As a result, power from the power supply unit 111C is supplied to the heating unit 121C of the heater assembly 30 via the main board 50.

### (Vibrating Device)

The vibrating device 60 comprises a vibrating element such as a vibrating motor. As shown in Figure 6, the vibrating device 60 is arranged between the upper surface of the power supply unit 111C and the upper wall section 403 in the power holding section 41 of the chassis 40. The lead wire 61 of the vibrating device 60 is connected to the peripheral FPC 72. The vibrating device 60 vibrates in a predetermined vibration mode based on a command from the MCU 1 to notify the user of predetermined information. For example, the vibrating device 60 vibrates in a predetermined vibration mode at the start or end of heating of the stick-type substrate 150 to notify the user of the start or end of heating. The vibrating device 60 is an example of the notification unit 113B of the inhalation device 100B shown in Figure 2.

### (Heater Assembly)

Figure 7 is a cross-sectional perspective view of the heater assembly 30.

The heater assembly 30 includes a heating unit 121C, an accommodating portion 140C, and a heat insulation section 144C. The heating unit 121C is, for example, a film heater and is wound around the outer circumference of the accommodating portion 140C. Additionally, the heating unit 121C and the board connection section 121a may be composed of a single heater FPC.

The heater assembly 30 is also provided with a stick guide 31. The stick guide 31 is provided at the top of the heater assembly 30 and guides the insertion and removal of the stick-type substrate 150 into and from the accommodating portion 140C. The stick guide 31 is a cylindrical member with an opening 27 and constitutes a part of the accommodating portion 140C.

The heater assembly 30 is also equipped with a heater temperature sensor 15 capable of detecting the temperature of the heating unit 121C. More specifically, the heater temperature sensor 15 is provided in contact with or in proximity to the heating unit 121C between the heating unit 121C and the heat insulation section 144C. The heater temperature sensor 15 is, for example, a thermistor.

### (Sensor FPC)

As shown in Figure 6, the sensor FPC 73 is arranged between the standing wall section 404 of the heater holding section 43 and the heater assembly 30. The sensor FPC 73 is equipped with a stick detection sensor 12, an inhalation sensor 13, and a case temperature sensor 14. The stick detection sensor 12, inhalation sensor 13, and case temperature sensor 14 are examples of the sensor unit 112B of the inhalation device 100B shown in Figure 2.

The stick detection sensor 12 is a sensor capable of detecting the stick-type substrate 150 accommodated in the accommodating portion 140C. In this embodiment, the stick detection sensor 12 is an optical sensor capable of detecting the stick-type substrate 150 based on the amount of reflected light from the light irradiated onto the accommodating portion 140C. Here, the amount of light includes concepts such as luminous flux, illuminance, luminous intensity, and brightness. The optical sensor is, for example, an IR (Infrared Ray) sensor.

The inhalation sensor 13 is a sensor that detects a puff operation (inhalation operation) by the user. The inhalation sensor 13 comprises, for example, a condenser microphone or a pressure sensor. The inhalation sensor 13 is provided near the stick guide 31 on the sensor FPC 73.

The case temperature sensor 14 is a sensor that detects the temperature of the case 20. The case temperature sensor 14 is, for example, a thermistor. The case temperature sensor 14 is arranged adjacent to the inner surface of the case 20 on the sensor FPC 73.

The sensor FPC 73 is also provided with a heater temperature sensor connection section 731 that connects to the heater temperature sensor 15 of the heater assembly 30. The heater temperature sensor connection section 731 is provided at the lower part of the sensor FPC 73. More specifically, the heater temperature sensor 15 is connected to a lead wire 15a, and the heater temperature sensor connection section 731 connects to the lead wire 15a extending from below the heater assembly 30.

The stick detection sensor 12, inhalation sensor 13, case temperature sensor 14, and heater temperature sensor connection section 731 are connected to the board connection section 730 via conductive tracks formed on the sensor FPC 73. The board connection section 730 is connected to the sensor FPC connection section 55 provided in the central region of the front surface 501 of the main board 50. As a result, the detection results of each sensor are output to the MCU 1 and other components mounted on the main board 50.

In the inhalation device 100 configured as described above, when the open state of the shutter 23 is detected by the shutter detection sensor 11 and the stick-type substrate 150 is detected by the stick detection sensor 12, the MCU 1 starts heating the heating unit 121C. When the user holds the mouthpiece section 152 of the stick-type substrate 150 in the mouth and inhales, aerosol from the aerosol source of the stick-type substrate 150 heated by the heating unit 121C is supplied into the user's mouth. The inhalation sensor 13 detects the number of inhalations, and the MCU 1 stops heating after a predetermined number of inhalations or after a predetermined time has elapsed. During heating of the inhalation device 100, the case temperature sensor 14, heater temperature sensor 15, and power temperature sensor 16 detect each temperature, and if abnormal heating is determined, the MCU 1 stops or suppresses heating of the heating unit 121C. Additionally, the user can operate the operation unit 24 to check the SOC of the power supply unit 111C, for example. The light-emitting unit 25 (LED 251) and the vibrating device 60 notify the user of various information such as the SOC of the power supply unit 111C and error displays. If the SOC of the power supply unit 111C decreases, the user can connect an external power source to the USB port 26 to charge the power supply unit 111C.

### [Stick Detection Sensor]

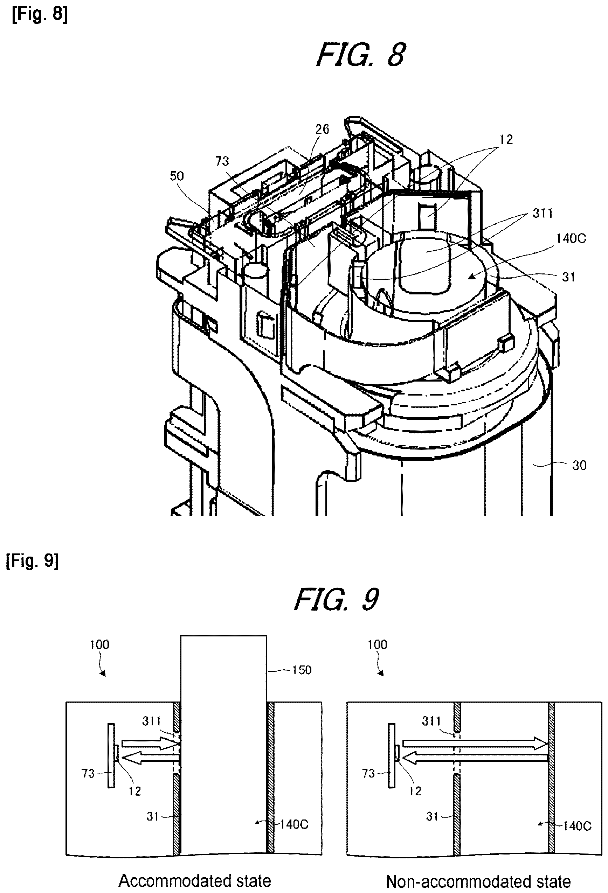

Next, the details of the stick detection sensor 12 will be described with reference to Figures 8 and 9.

The stick detection sensor 12 is an optical sensor that irradiates light onto the accommodating portion 140C and detects the amount of reflected light from the accommodating portion 140C. The MCU 1 is configured to detect whether the stick-type substrate 150 is accommodated in the accommodating portion 140C based on the amount of reflected light detected by the stick detection sensor 12. Here, the light irradiated and received by the stick detection sensor 12 is, for example, near-infrared light, and in this case, the stick detection sensor 12 is an IR sensor. In the following, the stick detection sensor 12 is assumed to detect "brightness" as an example of the amount of light.

Figure 8 is a cross-sectional view along line A-A in Figure 5, showing the structure around the sensor FPC 73, stick detection sensor 12, and stick guide 31 (accommodating portion 140C). The sensor FPC 73 is a flexible member arranged around the accommodating portion 140C. The stick detection sensor 12 is provided on the sensor FPC 73. As a result, the stick detection sensor 12 can be easily arranged around the accommodating portion 140C compared to when the stick detection sensor 12 is provided on the rigid main board 50. Since there is a high degree of freedom in arrangement, the size of the inhalation device 100 can be reduced.

The stick detection sensor 12 is arranged at a predetermined distance from the stick guide 31, reducing the influence of heat from the stick guide 31 (accommodating portion 140C). Additionally, a part of the wall section partitioning the accommodating portion 140C in the stick guide 31 is provided with a transparent filter 311 that allows light to pass through, and the sensor FPC 73 is arranged around the accommodating portion 140C so that the stick detection sensor 12 faces the transparent filter 311 at a predetermined distance. The part of the stick guide 31 not provided with the transparent filter 311 is configured to be impermeable to light.

As shown in Figure 9, the stick detection sensor 12 irradiates light onto the accommodating portion 140C through the transparent filter 311 and receives the reflected light. When the stick-type substrate 150 is accommodated in the accommodating portion 140C (hereinafter also referred to as the accommodated state), the light irradiated from the stick detection sensor 12 is immediately reflected on the surface of the stick-type substrate 150 after passing through the transparent filter 311. The stick detection sensor 12 receives the reflected light reflected on the surface of the stick-type substrate 150. On the other hand, when the stick-type substrate 150 is not accommodated in the accommodating portion 140C (hereinafter also referred to as the non-accommodated state), the light irradiated from the stick detection sensor 12 passes through the transparent filter 311, travels inside the accommodating portion 140C, and is reflected on the inner wall of the accommodating portion 140C. The stick detection sensor 12 receives the reflected light reflected on the inner wall of the accommodating portion 140C.

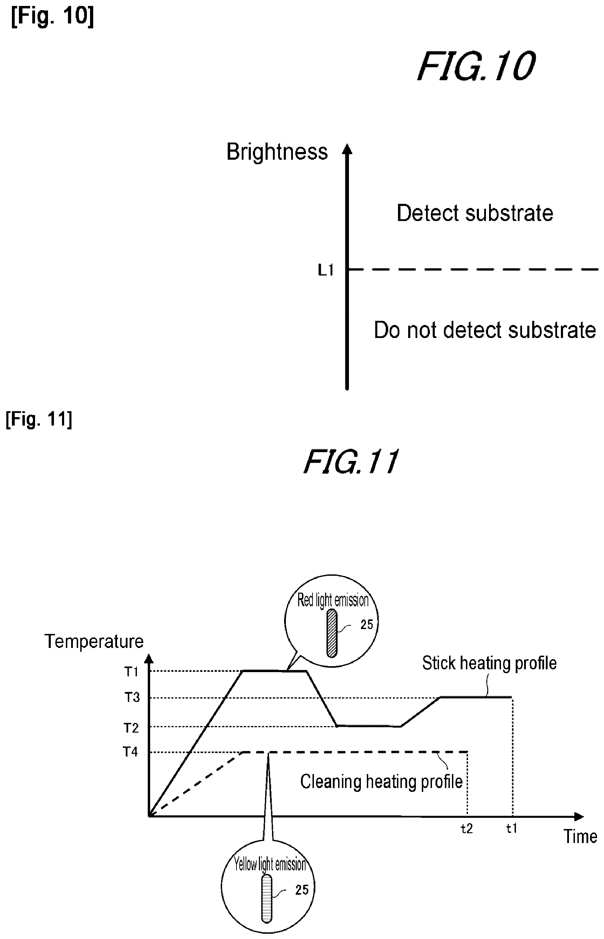

In this way, the distance the light travels from irradiation to reception in the accommodated state is shorter than in the non-accommodated state. Thus, the brightness of the reflected light received by the stick sensing sensor 12 in the received state is higher than in the unreceived state. Based on the difference in brightness between the accommodated state and the non-accommodated state, the MCU 1 detects the stick-type substrate 150. Specifically, as shown in Figure 10, the MCU 1 detects the stick-type substrate 150 when the brightness of the reflected light detected by the stick detection sensor 12 is equal to or greater than a predetermined value L1. On the other hand, the MCU 1 does not detect the stick-type substrate 150 when the brightness of the reflected light detected by the stick detection sensor 12 is less than the predetermined value L1.

In this embodiment, two stick detection sensors 12 and two transparent filters 311 are provided. For example, the MCU 1 can be configured not to detect the stick-type substrate 150 unless the detection results of both stick detection sensors 12 indicate the accommodated state of the stick-type substrate 150.

### [Example of Operation of the Inhalation Device]

Next, an example of the operation of the inhalation device 100 will be described.

The inhalation device 100 is activated, for example, in response to the shutter 23 being in the open state. Specifically, the MCU 1 is activated in response to the shutter detection sensor 11 detecting the open state of the shutter 23. After the activation of the MCU 1, the operation of the heating unit 121C becomes possible. Here, the shutter detection sensor 11 is composed of, for example, a magnet provided on the shutter 23 and a Hall IC (Integrated Circuit) provided at the upper end of the main board 50. Note that the MCU 1 may also be activated in response to the pressing of the operation unit 24.

Next, the automatic heating mode and manual heating mode as modes for starting the operation of the heating unit 121C will be described.

The automatic heating mode is a mode in which the operation of the heating unit 121C is automatically started in response to the stick-type substrate 150 being accommodated in the accommodating portion 140C. In the automatic heating mode, for example, in response to the shutter 23 being in the open state, the stick detection sensor 12 starts irradiating and receiving light and detects the amount of reflected light. When the automatic heating mode is selected, the MCU 1 starts heating the stick-type substrate 150 after detecting the stick-type substrate 150 based on the detection result of the stick detection sensor 12.

The manual heating mode is a mode in which the operation of the heating unit 121C is started in response to a heating request from the user. When the manual heating mode is selected, the MCU 1 does not automatically start heating the stick-type substrate 150 even if the stick-type substrate 150 is detected. The MCU 1 starts heating the stick-type substrate 150 in response to a heating request from the user. Here, the heating request from the user is, for example, the pressing of the operation unit 24 or an inhalation operation on the inhalation device 100.

The user selects either the automatic heating mode or the manual heating mode. The mode selection is performed, for example, on a user terminal (such as a smartphone), and the MCU 1 receives instruction information from the user terminal via the communication unit 115B and sets the mode selected by the user.

Next, the heating of the stick-type substrate 150 will be described.

When the stick-type substrate 150 is accommodated in the accommodating portion 140C, the MCU 1 operates the heating unit 121C based on the stick heating profile for heating the stick-type substrate 150. The stick heating profile is information that defines the time-series transition of the target temperature, which is the target value of the temperature of the heating unit 121C, and is information for heating the stick-type substrate 150. The stick heating profile is stored in advance in ROM, for example. The MCU 1 controls the temperature of the heating unit 121C based on the stick heating profile to generate aerosol from the stick-type substrate 150.

The solid line shown in Figure 11 is an example of the stick heating profile. According to the stick heating profile, the temperature of the heating unit 121C is raised to the maximum temperature T1 with the start of heating control, then lowered to T2, and then raised again to T3. When the elapsed time from the start of heating control reaches t1, the heating control is terminated. In Figure 11, when the temperature of the heating unit 121C is assumed to have reached T1 and the heating unit 121C is sufficiently hot, it is assumed that a sufficient amount of aerosol is generated, and the user can inhale. The heating period before inhalation becomes possible is also referred to as the preheating period.

To elaborate on the temperature control of the heating unit 121C based on the stick heating profile, the MCU 1 controls the temperature of the heating unit 121C based on the deviation between the target temperature corresponding to the elapsed time from the start of heating control and the actual temperature of the heating unit 121C (hereinafter also referred to as "actual temperature"). More specifically, at this time, the MCU 1 controls the temperature of the heating unit 121C so that the time-series transition of the actual temperature of the heating unit 121C becomes similar to the time-series transition of the target temperature defined in the stick heating profile. Note that the heating control of the accommodating portion 140C based on the cleaning heating profile described later is performed in the same manner.

The stick heating profile is typically designed to optimize the flavor experienced by the user when inhaling the aerosol generated from the stick-type substrate 150. Therefore, by controlling the temperature of the heating unit 121C based on the stick heating profile, the flavor experienced by the user can be optimized, providing the user with a high-quality smoking experience.

Next, the heating of the accommodating portion 140C when the stick-type substrate 150 is not accommodated in the accommodating portion 140C will be described.

The accommodating portion 140C may become dirty with the use of the inhalation device 100. For example, a part of the aerosol source of the stick-type substrate 150 (e.g., tobacco leaves) may spill into the accommodating portion 140C, or a part of the aerosol generated by heating the stick-type substrate 150 may become liquid and adhere to the accommodating portion 140C. Generally, when dirt adheres to the accommodating portion 140C, the quality of the flavor experienced by the user decreases, so it is desirable for the user to clean the accommodating portion 140C regularly.

The cleaning of the accommodating portion 140C is performed, for example, by inserting a cleaning tool (e.g., a cotton swab) with a cleaning agent (e.g., a liquid substance such as alcohol or water) into the accommodating portion 140C. This allows the removal of dirt adhering to the accommodating portion 140C. However, immediately after cleaning, the accommodating portion 140C remains wet with the cleaning agent, so if the user uses the inhalation device 100 while the inside of the accommodating portion 140C is wet immediately after cleaning, poor-quality smoking flavor aerosol may be delivered to the user.

Therefore, in this embodiment, when the stick-type substrate 150 is not accommodated in the accommodating portion 140C, the MCU 1 operates the heating unit 121C based on the cleaning heating profile for heating the accommodating portion 140C without the stick-type substrate 150. The cleaning heating profile is information that defines the time-series transition of the target temperature, which is the target value of the temperature of the heating unit 121C, and is information for cleaning the inside of the accommodating portion 140C. The cleaning heating profile is stored in advance in ROM, for example. The cleaning heating profile is a different heating profile from the stick heating profile, and, for example, the information such as the target temperature and operation time is different, as described later.

In this way, by controlling the temperature of the heating unit 121C based on the cleaning heating profile, the MCU 1 can evaporate and remove the cleaning agent adhering to the accommodating portion 140C without the stick-type substrate 150. Therefore, even immediately after the user cleans the accommodating portion 140C with a cleaning tool, the inhalation device 100 can provide a high-quality smoking experience to the user without degrading the quality of the flavor as a result of the MCU 1 heating the accommodating portion 140C based on the cleaning heating profile. Thus, the convenience of using the inhalation device 100 can be improved.

Additionally, the heating control based on the cleaning heating profile can remove not only the cleaning agent but also the dirt adhering to the accommodating portion 140C with the use of the inhalation device 100, as described above. Specifically, by the heating control based on the cleaning heating profile, the liquid present in the accommodating portion 140C evaporates, so a part of the aerosol source (e.g., tobacco leaves) that adhered to the accommodating portion 140C due to the liquid no longer adheres to the accommodating portion 140C. Therefore, the user can easily remove a part of the aerosol source from the accommodating portion 140C by, for example, directing the opening 27 downward.

Regarding the conditions for executing the heating control based on the cleaning heating profile, the MCU 1 operates the heating unit 121C based on the cleaning heating profile when there is a heating request from the user and the stick-type substrate 150 is not accommodated in the accommodating portion 140C. Here, the heating request from the user is, for example, the pressing of the operation unit 24. By making the heating request from the user a condition for the heating control based on the cleaning heating profile, the cleaning heating can be executed based on the user's intention.

Furthermore, in this embodiment, the MCU 1 may operate the heating unit 121C based on the cleaning heating profile on the condition that the manual heating mode is selected in addition to the above conditions.

In this embodiment, when the manual heating mode is selected, the stick detection sensor 12 starts operating (irradiating and receiving light) when there is a heating request from the user. The MCU 1 operates the heating unit 121C based on the cleaning heating profile when there is a heating request from the user and the stick-type substrate 150 is not detected based on the detection result of the stick detection sensor 12. By starting the operation of the stick detection sensor 12 when there is a heating request from the user, power consumption can be reduced compared to when the stick detection sensor 12 operates continuously. Note that the timing for the stick detection sensor 12 to start operating in the manual heating mode is not limited to this. Even in the manual heating mode, the stick detection sensor 12 may be configured to start operating in response to the shutter 23 being in the open state, similar to the automatic heating mode.

Here, with reference to Figure 11, the cleaning heating profile (dashed line in Figure 11) will be described in comparison with the stick heating profile.

According to the cleaning heating profile, the temperature of the heating unit 121C is raised to the maximum temperature T4 with the start of heating control and then maintained at that temperature T4. When the elapsed time from the start of heating control reaches t2, the heating control is terminated.

The target temperature of the heating unit 121C in the cleaning heating profile is set lower than the target temperature of the heating unit 121C in the stick heating profile. Specifically, while the maximum temperature T1 of the target temperature in the stick heating profile is set to about 300°C, the maximum temperature T4 of the target temperature in the cleaning heating profile is about 100°C to 200°C. The maximum temperature T4 of the cleaning heating profile is set to a temperature that can evaporate the moisture in the accommodating portion 140C, for example, above the boiling point of water. By setting the target temperature in this way, excessive heating of the accommodating portion 140C without the stick-type substrate 150 is suppressed.

Additionally, the stick heating profile and the cleaning heating profile include information on the operation time for operating the heating unit 121C, and the operation time t2 of the cleaning heating profile is set shorter than the operation time t1 of the stick heating profile. The operation time t2 of the cleaning heating profile should be sufficient to evaporate the moisture in the accommodating portion 140C. By setting the operation time in this way, excessive heating of the accommodating portion 140C without the stick-type substrate 150 is suppressed. Additionally, power consumption can be reduced.

### [Example of Notification by the Notification Unit]

Next, an example of notification to the user during heating will be described. Here, the light emission by the light-emitting unit 25 (LED 251), which is an example of the notification unit 113B in Figure 2, will be described.

The light-emitting unit 25 notifies the user that the heating unit 121C is in operation. Specifically, the light-emitting unit 25 emits light in a predetermined emission mode when the heating unit 121C is in operation while the stick-type substrate 150 is accommodated in the accommodating portion 140C (i.e., the accommodated state) and when the heating unit 121C is in operation while the stick-type substrate 150 is not accommodated in the accommodating portion 140C (i.e., the non-accommodated state). Such notification allows the user to easily visually recognize that the heating unit 121C is in operation. Particularly, when the heating unit 121C is in operation in the accommodated state, the user can confirm the light emission by the light-emitting unit 25 and, for example, be careful not to bring fingers close to the vicinity of the opening 27.

Additionally, the light-emitting unit 25 may emit light in different emission modes when the heating unit 121C is in operation in the accommodated state and when the heating unit 121C is in operation in the non-accommodated state. For example, as shown in Figure 11, the emission color of the LED 251 is changed between the emission mode during heating control based on the stick heating profile and the emission mode during heating control based on the cleaning heating profile. Additionally, the emission mode can be distinguished by changing the number of LEDs 251 among the plurality of LEDs 251 that emit light. By setting the emission modes differently between the accommodated state and the non-accommodated state, the user can easily visually recognize that different heating control is being performed when the heating unit 121C is in operation in the non-accommodated state compared to when it is in operation in the accommodated state.

The notification by the notification unit 113B is not limited to light emission by the light-emitting unit 25 and may be vibration by the vibrating device 60, for example. Specifically, the vibrating device 60 may vibrate during the operation of the heating unit 121C to notify the user that the heating unit 121C is in operation. Additionally, the vibrating device 60 may vibrate in different vibration modes when the heating unit 121C is in operation in the accommodated state and when the heating unit 121C is in operation in the non-accommodated state.

### [Example of Processing Executed by the Control Unit]

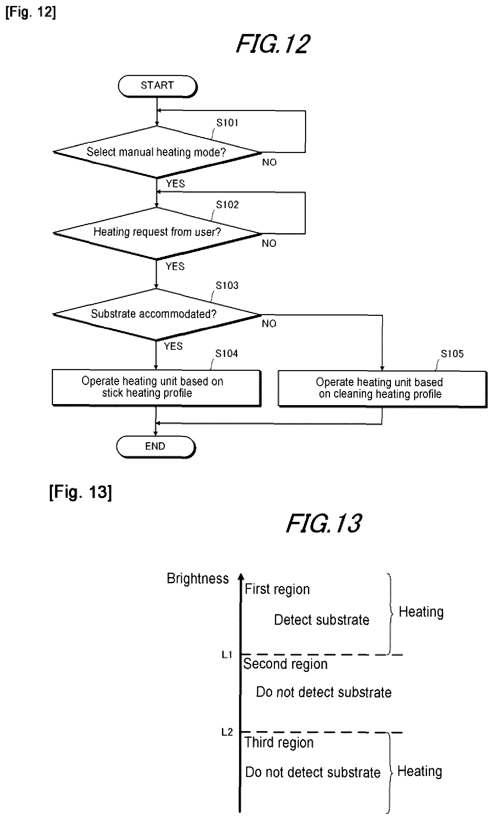

Next, an example of processing executed by the MCU 1 will be described using the flowchart shown in Figure 12.

The MCU 1 first determines whether the manual heating mode is selected (Step S101). If the manual heating mode is not selected (Step S101: NO), the MCU 1 repeatedly monitors Step S101 until the manual heating mode is selected.

If the manual heating mode is selected (Step S101: YES), the MCU 1 determines whether there is a heating request from the user (Step S102). If there is no heating request from the user (Step S102: NO), the MCU 1 repeatedly monitors Step S102 until there is a heating request from the user.

When there is a heating request from the user (Step S102: YES), the MCU 1 determines whether the stick-type substrate 150 is in the accommodated state in the accommodating portion 140C (Step S103). Specifically, when there is a heating request from the user, the stick detection sensor 12 starts operating, and the MCU 1 obtains the detection result from the stick detection sensor 12 to determine whether the stick-type substrate 150 is in the accommodated state in the accommodating portion 140C.

When the stick-type substrate 150 is in the accommodated state in the accommodating portion 140C (Step S103: YES), the MCU 1 operates the heating unit 121C based on the stick heating profile (Step S104). As a result, the heating of the stick-type substrate 150 is started, and aerosol is generated.

On the other hand, when the stick-type substrate 150 is not in the accommodated state in the accommodating portion 140C (Step S103: NO), the MCU 1 operates the heating unit 121C based on the cleaning heating profile (Step S105). As a result, the liquid substance in the accommodating portion 140C can be evaporated and removed by heating with the heating unit 121C.

In the embodiment described above, the MCU 1 operates the heating unit 121C based on the cleaning heating profile on the premise that the manual heating mode is selected (i.e., YES in Step S101), but this is not limited to this. For example, even when the automatic heating mode is selected, the heating unit 121C may be operated based on the cleaning heating profile. In such a configuration, for example, the MCU 1 may operate the heating unit 121C based on the cleaning heating profile when there is a heating request from the user (e.g., pressing the operation unit 24).

### <<Variant Examples>>

In the embodiment described above, when the brightness of the reflected light is less than the predetermined value L1, the MCU 1 does not detect the stick-type substrate 150 and operates the heating unit 121C based on the cleaning heating profile. This variant differs from the embodiment described above in that the MCU 1 may not operate the heating unit 121C even when the brightness of the reflected light is less than the predetermined value L1.

When there is a large amount of cleaning agent or dirt in the accommodating portion 140C due to the use of the inhalation device 100, the brightness of the reflected light may be smaller compared to when there is less cleaning agent or dirt. This is because the light irradiated from the stick detection sensor 12 is scattered by the cleaning agent or dirt when reflected on the inner wall of the accommodating portion 140C, reducing the amount of light returning to the stick detection sensor 12. In this modification, when it is determined based on the brightness of the reflected light that there is little cleaning agent or dirt in the accommodating portion 140C, the MCU 1 does not operate the heating unit 121C.

This variant will be described specifically. First, regarding the region of brightness of the reflected light detected by the stick detection sensor 12, as shown in Figure 13, when the brightness of the reflected light is included in the first region (region where brightness is equal to or greater than L1), the MCU 1 detects the stick-type substrate 150. Additionally, when the brightness of the reflected light is included in the second region (region where brightness is equal to or greater than L2 and less than L1) or the third region (region where brightness is less than L2), the MCU 1 does not detect the stick-type substrate 150. Here, L2 is a value smaller than L1. These regions are different from each other. The second region has little cleaning agent or dirt in the accommodating portion 140C, and the brightness of the reflected light is high, while the third region has a lot of cleaning agent or dirt in the accommodating portion 140C, and the brightness of the reflected light is low.

When the brightness detected by the stick detection sensor 12 is included in the first region, the MCU 1 detects the stick-type substrate 150 and operates the heating unit 121C based on the stick heating profile as described in the embodiment above.

Additionally, when the brightness is included in the second region, the MCU 1 does not detect the stick-type substrate 150 and does not operate the heating unit 121C. As a result, when it is determined that there is little cleaning agent or dirt in the accommodating portion 140C, the MCU 1 does not operate the heating unit 121C, reducing power consumption. Note that when a heating request from the user is a condition for heating control, the MCU 1 does not operate the heating unit 121C contrary to the heating request from the user, so the user may be notified that the heating unit 121C is not operated via the communication unit such as the light-emitting unit 25 or the vibrating device 60.

Additionally, when the brightness is included in the third region, the MCU 1 does not detect the stick-type substrate 150 and operates the heating unit 121C based on the cleaning heating profile. As a result, when it is determined that there is a lot of cleaning agent or dirt in the accommodating portion 140C, the heating unit 121C is operated based on the cleaning heating profile to evaporate and remove the liquid substance in the accommodating portion 140C.

In the case where the automatic heating mode is selected, as described above, the stick detection sensor 12 starts operating and detects the brightness of the reflected light in response to the shutter 23 being in the open state. When the brightness is included in the third region, the MCU 1 may prompt the user to perform heating based on the cleaning heating profile via the communication unit such as the light-emitting unit 25 or the vibrating device 60. As a result, the user who receives the notification can, for example, press the operation unit 24 to execute heating control based on the cleaning heating profile by the MCU 1. Additionally, in the case where the automatic heating mode is selected, the MCU 1 may operate the heating unit 121C based on the cleaning heating profile instead of prompting the user to perform heating based on the cleaning heating profile when the brightness is included in the third region.

The control method of the inhalation device 100 described in the embodiment and variant above can be realized by executing a pre-prepared program on a computer (processor). This program is stored in a computer-readable storage medium and executed by being read from the storage medium. Additionally, this program may be provided in a form stored in a non-transitory storage medium such as flash memory or may be provided via a network such as the internet. Additionally, the computer that executes this program can be, for example, one included in the inhalation device 100 (e.g., MCU 1), but is not limited to this and may be one included in another device (e.g., a smartphone or server device) that can communicate with the inhalation device 100.

The embodiments of the present invention have been described above with reference to the drawings, but it goes without saying that the present invention is not limited to such embodiments. It is apparent that those skilled in the art can conceive various variants or alterations within the scope described in the claims, and these are naturally understood to belong to the technical scope of the present invention. Additionally, the components in the embodiments described above may be combined arbitrarily without departing from the spirit of the invention.

For example, in the embodiment described above, an optical sensor is shown as an example of the stick detection sensor 12, but this is not limited to this. For example, the stick detection sensor 12 may be a pressure sensor that detects pressure fluctuations in the accommodating portion 140C accompanying the insertion and removal of the stick-type substrate 150. In this case, the MCU 1 detects the stick-type substrate 150 based on the pressure fluctuations detected by the pressure sensor. Additionally, if the stick-type substrate 150 is provided with identification information, the stick detection sensor 12 may be an identification information reader capable of reading the identification information of the stick-type substrate 150. In this case, the MCU 1 detects the stick-type substrate 150 based on the reading result by the identification information reader. Additionally, the stick detection sensor 12 may be a mechanical switch provided near the accommodating portion 140C (e.g., the bottom surface of the accommodating portion 140C) and pressed by the stick-type substrate 150. In this case, the MCU 1 detects the stick-type substrate 150 by the pressing of the switch. Additionally, if the stick-type substrate 150 includes a susceptor, the MCU 1 may detect the stick-type substrate 150 based on the change in the characteristics of the circuit of the inhalation device 100 (e.g., change in inductance) due to the insertion of the stick-type substrate 150.

The present specification etc. sets forth at least the following features. Corresponding components etc. in the embodiment described above are shown by way of example in parentheses, but are not limited thereto.
(1) An inhalation device (inhalation device 100, 100A, 100B) for generating aerosol from a substrate having an aerosol source (stick-type substrate 150), comprising an accommodating portion (accommodating portion 140, 140C) in which the substrate is accommodated, a heating unit (heating unit 121A to 121C) for heating the accommodating portion, and a control unit (control unit 116A, 116B, MCU 1) for controlling the heating unit based on heating information that defines a time-series transition of a target temperature, which is the target value of a temperature of the heating unit, wherein the heating information includes first heating information (stick heating profile) for heating the substrate and second heating information (cleaning heating profile) different from the first heating information, and the control unit operates the heating unit based on the first heating information when the substrate is accommodated in the accommodating portion and operates the heating unit based on the second heating information when the substrate is not accommodated in the accommodating portion.
   Dirt may adhere to the accommodating portion with the use of the inhalation device. When the user cleans the accommodating portion using a liquid such as alcohol or water to remove the dirt, the inside of the accommodating portion becomes wet immediately after cleaning. According to (1), the control unit operates the heating unit based on the second heating information different from the first heating information for heating the substrate when the substrate is not accommodated in the accommodating portion. This allows the evaporation and removal of liquids such as alcohol or water used for cleaning, so the user does not need to wait for the accommodating portion to dry naturally immediately after cleaning, and can enjoy a high-quality smoking experience even right after cleaning. Therefore, the convenience of using the inhalation device can be improved.
(2) The inhalation device according to (1), wherein the target temperature of the second heating information is lower than the target temperature of the first heating information.
   According to (2), when the heating unit is operated based on the second heating information, excessive heating of the accommodating portion without the substrate accommodated is suppressed.
(3) The inhalation device according to (2), wherein the maximum temperature of the target temperature of the second heating information is equal to or higher than the boiling point of water.
   According to (3), the heating of the accommodating portion based on the second heating information can evaporate the moisture inside the accommodating portion.
(4) The inhalation device according to any one of (1) to (3), wherein the first heating information and the second heating information each include an operation time for operating the heating unit, and the operation time of the second heating information is shorter than the operation time of the first heating information.
   According to (4), when the heating unit is operated based on the second heating information, excessive heating of the accommodating portion without the substrate accommodated is suppressed. Additionally, power consumption can be reduced.
(5) The inhalation device according to any one of (1) to (4), wherein the control unit operates the heating unit based on the second heating information when there is a heating request from the user and the substrate is not accommodated in the accommodating portion.
   According to (5), by making the heating request from the user a condition for heating control based on the second heating information, heating control based on the second heating information can be executed based on the user's intention.
(6) The inhalation device according to (5), wherein the control unit can selectively switch between a first mode (automatic heating mode) that automatically starts the operation of the heating unit in response to the substrate being accommodated in the accommodating portion, and a second mode (manual heating mode) that starts the operation of the heating unit in response to a heating request from the user, and when the second mode is selected, the control unit operates the heating unit based on the second heating information when there is a heating request from the user and the substrate is not accommodated in the accommodating portion.
   According to (6), since the operation of the heating unit can be selectively switched between the first mode and the second mode, the user can set the mode of heating operation according to their preference. Additionally, when the second mode is selected, the heating unit can be operated based on the second heating information when there is a heating request from the user.
(7) The inhalation device according to any one of (1) to (6), further comprising an optical sensor (stick detection sensor 12) that irradiates light onto the accommodating portion and detects the amount of reflected light from the accommodating portion, wherein the control unit is configured to detect the substrate based on the amount of reflected light.
   According to (7), it is possible to detect whether the substrate is accommodated in the accommodating portion based on the amount of light detected by the optical sensor.
(8) The inhalation device according to (7), wherein the control unit detects the substrate and operates the heating unit based on the first heating information when the amount of light is included in the first region, does not detect the substrate and does not operate the heating unit when the amount of light is included in a second region different from the first region, and does not detect the substrate and operates the heating unit based on the second heating information when the amount of light is included in a third region different from the first region and the second region.
   According to (8), since the heating unit is not operated when heating based on the second heating information is not necessary, power consumption can be reduced.
(9) The inhalation device according to (7) or (8), further comprising a flexible member (sensor FPC 73) arranged around the accommodating portion and electrically connected to the control unit, wherein the optical sensor is provided on the flexible member.
   According to (9), by providing the optical sensor on the flexible member, the degree of freedom in arranging the optical sensor around the accommodating portion is increased compared to when the optical sensor is provided on a rigid board.
(10) The inhalation device according to (9), wherein a part of the wall section partitioning the accommodating portion is provided with a light-transmissive member (transparent filter 311), and the flexible member is arranged around the accommodating portion so that the optical sensor faces the light-transmissive member at a predetermined distance.
   According to (10), since the optical sensor faces the light-transmissive member at a predetermined distance, the influence of heat from the accommodating portion on the optical sensor can be reduced.
(11) The inhalation device according to any one of (1) to (10), further comprising a notification unit (light-emitting unit 25, vibrating device 60) that notifies the user that the heating unit is in operation, wherein the notification unit notifies the user that the heating unit is in operation when the heating unit is in operation while the substrate is not accommodated in the accommodating portion.
   According to (11), the user can easily recognize that the heating unit is in operation while the substrate is not accommodated in the accommodating portion and can be careful not to bring fingers close to the vicinity of the accommodating portion.
(12) The inhalation device according to (11), wherein the notification unit notifies the user that the heating unit is in operation in a first notification mode when the heating unit is in operation while the substrate is accommodated in the accommodating portion, and notifies the user that the heating unit is in operation in a second notification mode different from the first notification mode when the heating unit is in operation while the substrate is not accommodated in the accommodating portion.
   According to (12), when the heating unit is in operation while the substrate is not accommodated in the accommodating portion, the user can easily recognize that different heating control is being performed compared to when the heating unit is in operation while the substrate is accommodated in the accommodating portion.
(13) The inhalation device according to (12), wherein the notification unit includes a light-emitting unit (light-emitting unit 25) that notifies the user by light emission, and the first notification mode and the second notification mode have different emission modes.
   According to (13), the user can easily visually recognize whether heating control based on the first heating information is being performed or heating control based on the second heating information is being performed.
(14) A control method executed by a computer (control unit 116A, 116B, MCU 1) for controlling the operation of an inhalation device (inhalation device 100, 100A, 100B) that generates aerosol from a substrate having an aerosol source (stick-type substrate 150), wherein the inhalation device includes an accommodating portion (accommodating portion 140, 140C) in which the substrate is accommodated and a heating unit (heating unit 121A to 121C) for heating the accommodating portion, and the computer is configured to control the heating unit based on heating information that defines the time-series transition of a target temperature, which is the target value of a temperature of the heating unit, wherein the heating information includes first heating information (stick heating profile) for heating the substrate and second heating information (cleaning heating profile) different from the first heating information, and the computer operates the heating unit based on the first heating information when the substrate is accommodated in the accommodating portion and operates the heating unit based on the second heating information when the substrate is not accommodated in the accommodating portion.
   Dirt may adhere to the accommodating portion with the use of the inhalation device. When the user cleans the accommodating portion using a liquid such as alcohol or water to remove the dirt, the inside of the accommodating portion becomes wet immediately after cleaning. According to (14), the computer operates the heating unit based on the second heating information, which is different from the first heating information for heating the substrate, when the substrate is not accommodated in the accommodating portion. This allows the evaporation and removal of liquids such as alcohol or water used for cleaning, so the user does not need to wait for the accommodating portion to dry naturally immediately after cleaning, and can enjoy a high-quality smoking experience even right after cleaning. Therefore, the convenience of using the inhalation device can be improved.
(15) A program for causing a computer (control unit 116A, 116B, MCU 1) for controlling the operation of an inhalation device (inhalation device 100, 100A, 100B) that generates aerosol from a substrate having an aerosol source (stick-type substrate 150) to execute predetermined processing, wherein the inhalation device includes an accommodating portion (accommodating portion 140C) in which the substrate is accommodated and a heating unit (heating unit 121A to 121C) for heating the accommodating portion, and the computer is configured to control the heating unit based on heating information that defines the time-series transition of a target temperature, which is the target value of a temperature of the heating unit, wherein the heating information includes first heating information (stick heating profile) for heating the substrate and second heating information (cleaning heating profile) different from the first heating information, and the program causes the computer to execute processing for operating the heating unit based on the first heating information when the substrate is accommodated in the accommodating portion and operating the heating unit based on the second heating information when the substrate is not accommodated in the accommodating portion.
   Dirt may adhere to the accommodating portion with the use of the inhalation device. When the user cleans the accommodating portion using a liquid such as alcohol or water to remove the dirt, the inside of the accommodating portion becomes wet immediately after cleaning. According to (15), the computer operates the heating unit based on the second heating information, which is different from the first heating information for heating the substrate, when the substrate is not accommodated in the accommodating portion. This allows the evaporation and removal of liquids such as alcohol or water used for cleaning, so the user does not need to wait for the accommodating portion to dry naturally immediately after cleaning, and can enjoy a high-quality smoking experience even right after cleaning. Therefore, the convenience of using the inhalation device can be improved.
(16) An inhalation system comprising a substrate having an aerosol source (stick-type substrate 150) and the inhalation device (inhalation device 100, 100A, 100B) according to any one of (1) to (13).
   Dirt may adhere to the accommodating portion with the use of the inhalation device. When the user cleans the accommodating portion using a liquid such as alcohol or water to remove the dirt, the inside of the accommodating portion becomes wet immediately after cleaning. According to (16), the control unit operates the heating unit based on the second heating information different from the first heating information for heating the substrate when the substrate is not accommodated in the accommodating portion. This allows the evaporation and removal of liquids such as alcohol or water used for cleaning, so the user does not need to wait for the accommodating portion to dry naturally immediately after cleaning, and can enjoy a high-quality smoking experience even right after cleaning. Therefore, the convenience of using the inhalation device can be improved.
(17) The inhalation device according to (13), wherein the emission color of the light-emitting unit differs between the first notification mode and the second notification mode.
   According to (17), the user can easily recognize whether heating control based on the first heating information or heating control based on the second heating information is being performed by checking the emission color.
(18) The inhalation device according to (13), wherein the light-emitting unit includes multiple light-emitting elements (LED 251), and the number of light-emitting elements that emit light differs between the first notification mode and the second notification mode.

According to (18), the user can easily recognize whether heating control based on the first heating information or heating control based on the second heating information is being performed by checking the number of light-emitting elements that emit light.

### REFERENCE SIGNS LIST

1 MCU (control unit, computer)
12 Stick detection sensor (optical sensor)
25 Light-emitting unit (notification unit)
60 Vibrating device (notification unit)
73 Sensor FPC (flexible member)
100, 100A, 100B Inhalation device
116A, 116B Control unit (computer)
121A to 121C Heating unit
140, 140C Accommodating portion
150 Stick-type substrate (substrate)
311 Transparent filter (transmissive member)

## Claims

1. An inhalation device for generating aerosol from a substrate having an aerosol source, comprising an accommodating portion in which the substrate is accommodated, a heating unit for heating the accommodating portion, and a control unit for controlling the heating unit based on heating information that defines a time-series transition of a target temperature, which is the target value of a temperature of the heating unit, wherein the heating information includes first heating information for heating the substrate and second heating information different from the first heating information, and the control unit operates the heating unit based on the first heating information when the substrate is accommodated in the accommodating portion and operates the heating unit based on the second heating information when the substrate is not accommodated in the accommodating portion.

2. The inhalation device according to claim 1, wherein the target temperature of the second heating information is lower than the target temperature of the first heating information.

3. The inhalation device according to claim 2, wherein the maximum temperature of the target temperature of the second heating information is equal to or higher than the boiling point of water.

4. The inhalation device according to any one of claims 1 to 3, wherein the first heating information and the second heating information each include an operation time for operating the heating unit, and the operation time of the second heating information is shorter than the operation time of the first heating information.

5. The inhalation device according to any one of claims 1 to 4, wherein the control unit operates the heating unit based on the second heating information when there is a heating request from the user and the substrate is not accommodated in the accommodating portion.

6. The inhalation device according to claim 5, wherein the control unit can selectively switch between a first mode that automatically starts the operation of the heating unit in response to the substrate being accommodated in the accommodating portion and a second mode that starts the operation of the heating unit in response to a heating request from the user, and when the second mode is selected, the control unit operates the heating unit based on the second heating information when there is a heating request from the user and the substrate is not accommodated in the accommodating portion.

7. The inhalation device according to any one of claims 1 to 6, further comprising an optical sensor that irradiates light onto the accommodating portion and detects the amount of reflected light from the accommodating portion, wherein the control unit is configured to detect the substrate based on the amount of reflected light.

8. The inhalation device according to claim 7, wherein the control unit detects the substrate and operates the heating unit based on the first heating information when the amount of light is included in the first region, does not detect the substrate and does not operate the heating unit when the amount of light is included in a second region different from the first region, and does not detect the substrate and operates the heating unit based on the second heating information when the amount of light is included in a third region different from the first region and the second region.

9. The inhalation device according to claim 7 or 8, further comprising a flexible member arranged around the accommodating portion and electrically connected to the control unit, wherein the optical sensor is provided on the flexible member.

10. The inhalation device according to claim 9, wherein a part of the wall section partitioning the accommodating portion is provided with a light-transmissive member, and the flexible member is arranged around the accommodating portion so that the optical sensor faces the light-transmissive member at a predetermined distance.

11. The inhalation device according to any one of claims 1 to 10, further comprising a notification unit that notifies the user that the heating unit is in operation, wherein the notification unit notifies the user that the heating unit is in operation when the heating unit is in operation while the substrate is not accommodated in the accommodating portion.

12. The inhalation device according to claim 11, wherein the notification unit notifies the user that the heating unit is in operation in a first notification mode when the heating unit is in operation while the substrate is accommodated in the accommodating portion, and notifies the user that the heating unit is in operation in a second notification mode different from the first notification mode when the heating unit is in operation while the substrate is not accommodated in the accommodating portion.

13. The inhalation device according to claim 12, wherein the notification unit includes a light-emitting unit that notifies the user by light emission, and the first notification mode and the second notification mode have different light-emission modes.

14. A control method executed by a computer for controlling the operation of an inhalation device that generates aerosol from a substrate having an aerosol source, wherein the inhalation device includes an accommodating portion in which the substrate is accommodated and a heating unit for heating the accommodating portion, and the computer is configured to control the heating unit based on heating information that defines a time-series transition of a target temperature, which is the target value of a temperature of the heating unit, wherein the heating information includes first heating information for heating the substrate and second heating information different from the first heating information, and the computer operates the heating unit based on the first heating information when the substrate is accommodated in the accommodating portion and operates the heating unit based on the second heating information when the substrate is not accommodated in the accommodating portion.

15. A program for causing a computer for controlling the operation of an inhalation device that generates aerosol from a substrate having an aerosol source to execute predetermined processing, wherein the inhalation device includes an accommodating portion in which the substrate is accommodated and a heating unit for heating the accommodating portion, and the computer is configured to control the heating unit based on heating information that defines a time-series transition of a target temperature, which is the target value of a temperature of the heating unit, wherein the heating information includes first heating information for heating the substrate and second heating information different from the first heating information, and the program causes the computer to execute processing for operating the heating unit based on the first heating information when the substrate is accommodated in the accommodating portion and operating the heating unit based on the second heating information when the substrate is not accommodated in the accommodating portion.
